# EUROPEAN PATENT SPECIFICATION

(11) **EP 2 688 563 B1**
(45) Date of publication and mention of the grant of the patent: **15.03.2017**
(21) Application number: 12719508.9
(22) Date of filing: 23.03.2012
(51) Int. Cl.: A61K 31/00, A61K 38/05, C07C 59/00, C07C 323/58, C07D 213/00, C07D 233/00, C07D 307/00, C07H 17/00, C07K 5/06, A61P 35/00, A61P 19/08, A61P 19/02, A61P 31/04

(54) **GALLIUM COMPLEXES, PHARMACEUTICAL COMPOSITIONS AND METHODS OF USE**
GALLIUMKOMPLEXE, PHARMAZEUTISCHE ZUSAMMENSETZUNGEN UND ANWENDUNGSVERFAHREN
COMPLEXES DE GALLIUM, COMPOSITIONS PHARMACEUTIQUES ET PROCÉDÉS D'UTILISATION

(30) Priority: 24.03.2011 US 201161467170 P; 05.08.2011 US 201161515462 P; 22.03.2012 US 201213426877
(43) Date of publication of application: 29.01.2014
(73) Proprietor: Odonate Therapeutics, LLC, San Diego CA 92121 (US)
(72) Inventor: THOTTATHIL, John K., Mundelein Illinois 60060 (US); WARRELL, Raymond P., Jr., Westfield New Jersey 07090 (US)
(74) Representative: Vossius & Partner Patentanwälte Rechtsanwälte mbB
(86) International application number: PCT/US2012/030267
(87) International publication number: WO 2012/129473

(56) References cited:
- US-A- 5 258 376
- MARTIN M. FINNEGAN ET AL: "Neutral water-soluble post-transition-metal chelate complexes of medical interest: aluminum and gallium tris(3-hydroxy-4-pyronates)", INORGANIC CHEMISTRY, vol. 26, no. 13, 1 July 1987 (1987-07-01), pages 2171-2176, XP55028838, ISSN: 0020-1669, DOI: 10.1021/ic00260a033
- TAMMY G. LUTZ ET AL: "Metal chelation with natural products: isomaltol complexes of aluminum, gallium, and indium", INORGANIC CHEMISTRY, vol. 28, no. 4, 1 February 1989 (1989-02-01), pages 715-719, XP55028840, ISSN: 0020-1669, DOI: 10.1021/ic00303a021

## Description

### TECHNICAL FIELD

The present invention relates generally to complexes of gallium with a ligand and pharmaceutical gallium compositions comprising the complexes, in particular those compositions suitable for therapeutic oral administration.

### BACKGROUND OF THE INVENTION

Gallium has demonstrated pharmaceutical value for the treatment of many human and animal disorders, including hypercalcemia, cancer, metastatic bone disease, and especially certain widespread degenerative or metabolic bone diseases such as osteoporosis and Paget's disease, arthritis and autoimmune diseases. For example, numerous clinical studies have shown gallium to have antineoplastic activity, as well as the ability to reduce abnormally high bone turnover in Paget's disease (reviewed in Bernstein, Therapeutic Gallium Compounds, in Metallotherapeutic Drugs and Meta-Based Diagnostic Agents: The Use of Metals in Medicine 259-277 (Gielen and Tiekink eds., 2005)). Based on original discoveries by one of the co-inventors, gallium is currently approved for use in the United States as a gallium nitrate (Ga(NO₃)₃●9H₂O) solution for intravenous infusion (Ganite^{®}) to treat hypercalcemia of malignancy (Warrell RP Jr., et al., Gallium nitrate inhibits calcium resorption from bone and is effective treatment for cancer-related hypercalcemia. J. Clin. Invest. 73:1487-1490, 1984).

Finnegan et al., Inorg. Chem., 1987, 26(13), 2171-6 discloses tris(kojato)gallium(III) (M(ka)3) and suggests to study its cytotoxicity. Lutz et al., Inorg. Chem.,1989, 28(4), 715-9 discloses tris(isomaltolato)gallium(III).

More recently gallium has been studied for treatment of intracellular bacterial infections such as tuberculosis and chronic pulmonary infections caused by *P. aeruginosa,* for example as described in U.S. Patent Nos. 6203822 and 5997912. There is currently interest in developing gallium for the treatment of bacterial infections that are resistant to conventional antibiotics by mechanisms that include but are not limited to biofilm disruption. One of the co-inventors has reported the first human use of intravenous gallium for a patient with cystic fibrosis and severe infectious pneumonia, which demonstrated highly favorable pharmacokinetic results and high concentrations in infected sputum (Novick S, Dupont LJ, Baert Y, Warrell RP, Jr. Systemic gallium nitrate therapy results in sustained sputum concentrations that may be therapeutic for biofilm-associated pulmonary bacterial infections. In: Proceedings of the 101st Annual Meeting of the American Association for Cancer Research; 2010 Apr 17-21; Washington, DC. Philadelphia (PA): AACR; 2010. Abstract).

In spite of its established utility, however, the use of gallium in the treatment of such diseases is hampered by the fact that simple forms of gallium, such as gallium salts, lack high bioavailability when delivered orally. The low oral bioavailability of these gallium forms requires that either impractically large doses of orally delivered gallium be administered to the patient or that the gallium be administered via non-oral means (e.g., intravenous, intramuscular or subcutaneous delivery). At present, the repeated or chronic administration via the oral route of such gallium salts, in particular the chloride (halogen), nitrate, sulfate, etc. salts, is not believed to be practical with chronic conditions such as osteoporosis, metastatic bone disease, arthritis, autoimmune diseases, various infectious diseases, and Paget's disease due to their low bioavailability, lack of pharmaceutical acceptability or both. The low oral bioavailability of gallium salts is impractical due to the need for absorption of large doses of gallium (as much as 25-50 mg/day for treatment of chronic diseases) to achieve therapeutic effectiveness.

Efforts have been made to increase the bioavailability of orally administered gallium, particularly through chemical complexing. Several gallium complexes have been identified that demonstrate increased oral bioavailability, including, e.g., gallium maltolate (see, e.g., Bernstein et al., Metal-Based Drugs 7:33-47 (2000); U.S. Patent Nos. 5,258,376; 5,574,027; 5,883,088; 5,968,922; 5,998,397; 6,004,951; 6,048,851; 6,087,354)) and gallium 8-quinolinolate (see, e.g., Collery et al., Anticancer Res. 16:687-692 (1996); U.S. Patent No. 5,525,598; European Patent No. EP 0 599 881; International Application No. PCT/EP92/01687). Other therapeutic gallium complexes are described in, e.g., Arion et al., J. Inorg. Biochem. 91:298-305 (2002); Chitambar et al., Clin. Cancer Res. 2:1009-1015 (1996); Stojilkovic et al., Mol. Microbiol. 31:429-442 (1999); U.S. Patent Nos. 5,196,412; 5,281,578; and International Application No. PCT/US91/03599.

In this regard, the results of a recent Phase I study of a tablet formulation of gallium nitrate showed limited absorption following oral administration in human subjects. Nitrates, however, are not preferred as bacteria in the oral cavity and alimentary tract can reduce nitrates to nitrites, which has been implicated in certain ailments, including cancer. In addition, nitrates can induce serious drops in blood pressure (i.e., hypotension), particular in combination with certain drugs (e.g., sildenafil).

Thus, there is a continuing need for the development of new gallium complexes and pharmaceutical compositions, particularly those having enhanced oral bioavailablity and a high ratio of gallium to ligand in the complex with a low molecular weight. In addition, it is desirable to have reduced levels of potentially toxic counter ions, such as nitrate, in the complexes and compositions to reduce toxicity. Counterions without significant toxicity concerns such as chloride, oxide, hydroxide, iodide, bromide, sulfate, citrate or mixed salts may be acceptable in the complexes.

### SUMMARY

In one or more embodiments, the present invention relates to complexes of ionic gallium salts with ligands, wherein the complexes exhibit increased oral bioavailability as compared to the oral bioavailability of gallium salts. Having conducted considerable experimental investigation, however, it has been found that it is not possible to predict either bioavailability or bioactivity of these complexes despite having carefully synthesized, characterized, and analyzed both *in vitro* and *in vivo* properties of each complex. The ligands in these embodiments are selected from the group consisting of naturally and non-naturally occurring amino acids and their derivatives; mono- and polycarboxylic acids and their derivatives; piperidinic acid and N-acyl piperidinic acids; thio compounds; aromatic, aliphatic and heterocyclic α-hydroxy acid;, α-hydroxypyrones; phenols and phenolic acids, and; keto acids.

In one specific embodiment of the gallium-ligand complex the ligand is an amino acid. The amino acid ligand may be an α or a β amino acid, including naturally occurring and non-naturally occurring analogs of α and β amino acids. For example, the amino acid ligand in the gallium-ligand complex may be histidine, carnosine or methionine.

In another specific embodiment of the gallium-ligand complex, the ligand is a carboxylic acid or a carboxylic acid derivative including mono and poly, aliphatic, aromatic and heterocyclic carboxylic acids, including pyridine compounds such as picolinic acid (C₆H₅NO₂, pyridine-2-carboxylic acid).

In another specific embodiment of the gallium-ligand complex, the ligand is an aromatic, aliphatic or heterocyclic α-hydroxy acid, including mandelic acid (C₆H₅CH(OH)CO₂H, 2-Hydroxy-2-phenylacetic acid).

In another specific embodiment of the gallium-ligand complex, the ligand is an α-hydroxypyrone, including kojic acid (C₆H₆O₄; 5-hydroxy-2-(hydroxymethyl)-4-pyrone), hydroxymethyl-protected kojic acid and isomaltol.

In a further specific embodiment, the ligand of the gallium-ligand complex is selected from the group consisting of histidine, methionine, picolinic acid, mandelic acid, kojic acid, hydroxymethyl-protected kojic acid, isomaltol and carnosine.

In another embodiment, the present invention relates to pharmaceutical compositions comprising the foregoing complexes of ionic gallium with ligands and at least one pharmaceutically acceptable excipient. In a specific embodiment the pharmaceutical composition may be formulated for oral administration to a mammalian subject, particularly to a human patient in need of treatment for a disease or condition which is treatable with gallium. The pharmaceutical compositions may be formulated in oral dosage forms such as capsules, tablets, caplets and the like. The gallium-ligand complexes of the pharmaceutical composition may be prepared prior to formulation of a selected dosage form, or the gallium-complex may be allowed to form *in situ* during the formulation process after separate administration of the gallium salt and the ligand to the formulation matrix.

In yet another embodiment, the present invention relates to use of the foregoing gallium-ligand complexes as a medicament and to methods of treating a disease or condition which is treatable with gallium comprising administration of the gallium-ligand complexes to a mammalian subject, such as a human patient or a mammalian animal. An example of a disease or condition treatable with gallium is excessive bone resorption, such as occurs in osteoporosis, Paget's disease and certain cancers. In a specific embodiment, the use of the medicament is for treating a bacterial infection or the method of use is for treating a bacterial infection, either alone or further including administration of an antibiotic.

In a still further embodiment, the present invention relates to methods for making complexes of ionic gallium and a ligand, wherein the ligand is selected from the group consisting of picolinic acid, mandelic acid (including both R(-) and S(+) forms), kojic acid, hydroxymethyl-protected kojic acid, isomaltol, histidine, methionine and carnosine. Such methods comprise providing a solution of the ligand in water, alcohol or other suitable organic solvents such as methanol, chloroform, DMF and the like with a minimum amount of water at a suitable temperature (about -20 °C to 100 °C), adding solid or a solution of gallium salt in water or other suitable solvents to the ligands solution in portions, allowing the reaction between ionic gallium and the ligand to occur such that complexes are formed, and isolating the complexes. These reactions may or may not be conducted in the presence of basic reagents such as carbonates, bicarbonates, and hydroxides of alkali and alkaline earth metals. At times the reaction may require basic reagents such as sodium carbonate, sodium bicarbonate, etc. for the reaction to proceed to completion. Optionally, the isolated complexes may be washed with water to remove residual free gallium and gallium counterion and/or extracted with a suitable organic solvent and washed with water followed by evaporation of the solvent. The gallium complexes may further be purified by crystallization from a suitable solvent such as water, alcohols and/or a mixture thereof.

The gallium organic compounds of the invention are in an approximate 3:1 ratio of organic ligand:gallium. They may be salts, coordination complexes or a combination of a salt and a coordination complex. They are typically neutral compounds, but ionic compounds have also been made and are included in the invention.

### BRIEF DESCRIPTION OF THE DRAWINGS

Fig. 1 illustrates a general scheme for the preparation of gallium complexes.
Fig. 2 illustrates the oral bioavailability in dogs of preferred gallium complexes, expressed as concentration of gallium in blood (plasma) over time.

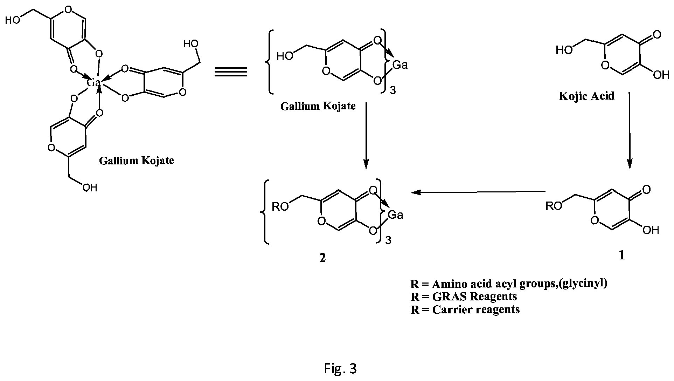

Fig. 3 illustrates two alternative pathways for preparation of a gallium kojate complex wherein the hydroxymethyl group of kojic acid is derivatized with an R group.

### DETAILED DESCRIPTION

Before describing several exemplary embodiments of the invention, it is to be understood that the invention is not limited to the details of construction or process steps set forth in the following description. The invention is capable of other embodiments and of being practiced or being carried out in various ways.

"Patient" includes both humans and other mammals.

The term "treating" or "treatment" of a state, disorder, disease or condition as used herein means: (1) preventing or delaying the appearance of clinical symptoms of the state, disorder, disease or condition developing in a mammal that may be afflicted with or predisposed to the state, disorder, disease or condition but does not yet experience or display clinical or subclinical symptoms of the state, disorder or condition, (2) inhibiting the state, disorder, disease or condition, i.e., arresting or reducing the development of the disease or at least one clinical or subclinical symptom thereof, or (3) relieving the disease, i.e., causing regression of the state, disorder or condition or at least one of its clinical or subclinical symptoms. The benefit to a subject to be treated is either statistically significant or at least perceptible to the patient and/or to the physician.

"Effective amount" and "therapeutically effective amount" mean the amount of a compound that, when administered to a mammal for treating a state, disorder, disease or condition, is sufficient to effect such treatment. The effective amount or therapeutically effective amount will vary depending on the compound, the disease and its severity, and the age, weight, physical condition and responsiveness of the individual to be treated.

"Delivering" and "administering" means providing a therapeutically effective amount of an active ingredient to a particular location or locations within a host causing a therapeutically effective blood concentration of the active ingredient at the particular location or locations. This can be accomplished by systemic administration of the active ingredient to the host, or by achieving high local concentrations (e.g., by installation into specific body cavities, by topical external application) that may avoid requirements for high systemic exposure.

"Pharmaceutically acceptable" means those active agents, salts and esters, and excipients which are, within the scope of sound medical judgment, suitable for use in contact with the tissues of humans and lower animals without undue toxicity, irritation, allergic response and the like, commensurate with a reasonable benefit/risk ratio, and effective for their intended use.

"Amino acid" is used herein in its broadest sense to mean the class of chemicals containing an amine group, a carboxylic acid group and a side-chain. This class includes both natural amino acids (i.e., those naturally incorporated into polypeptides) and non-standard amino acids (i.e., those either not found in proteins or not produced by cellular machinery).

In one or more embodiments, the present invention relates to complexes of ionic gallium salts with ligands, wherein the complexes exhibit increased oral bioavailability compared to gallium salts. In one or more specific embodiments, the ligand is a naturally occurring or non-naturally occurring amino acid or amino acid derivative, a carboxylic acid or carboxylic acid derivative, piperidinic acid or an N-acyl piperidinic acid, a thio compound, an aromatic, aliphatic or heterocyclic α-hydroxy acid, an α-hydroxypyrone, a phenol or phenolic acid, or a keto acid, including the specific embodiments of these compounds identified above. The gallium-ligand complexes are ideally characterized by a molecular ratio of ligand to gallium of about 3:1, indicating formation of a bidentate coordination complex salt.

In addition, the gallium-ligand complexes most suitable for pharmaceutical use contain substantially no residual undesirable counterion from the gallium salt used to prepare the complex, such as nitrate. In certain embodiments, the residual counter ion in the gallium-ligand complex preparation (for example, nitrate when gallium nitrate is the salt used) is no more than about 5%, about 0.3 to 5%, about 0.3 to 2% or about 0.3 to 1%. However, it is to be understood that higher amounts of gallium counterion are acceptable where the counterion has relatively low toxicity when the gallium-ligand complex is used as a therapeutic agent. The gallium-ligand complexes also preferably have low levels of residual counter ion from any basic reagents used. In this case the counterion (such as sodium) may be present in the gallium-ligand complex preparation in amounts less than about 10%, or between 0.1 and 2%.

In a first specific example of therapeutic use of the complexes of the invention, the gallium-ligand complex is a complex of ionic gallium with picolinic acid (C₁₈H₁₂GaN₃O₆). A proposed structure for this complex may be represented by structural Formula (I):

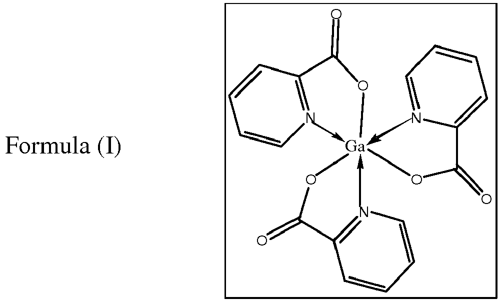

The gallium picolinate complex is a neutral complex with a molecular weight of 436, which supports a conclusion of formation of a 3:1 complex. The gallium picolinate complex contains about 10-20% by weight of gallium, about 12-18% gallium or about 16% gallium, resulting in a particularly high proportion of gallium relative to the molecular weight of the complex.

In a second specific example of therapeutic use of the complexes of the invention, the gallium-ligand complex is a complex of ionic gallium with kojic acid (C₁₈H₁₅GaO₁₂). A proposed structure for this complex may be represented by structural Formula (II):

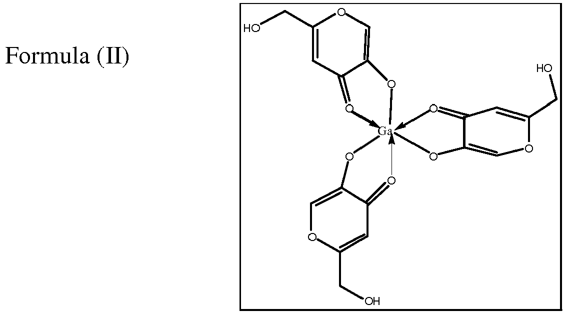

The gallium kojate complex is a neutral complex with a molecular weight of 493.02, which supports a conclusion of formation of a 3:1 complex. The gallium kojate complex contains about 10-18% gallium, about 12-16% gallium or about 15% gallium, resulting in a high proportion of gallium relative to the molecular weight of the complex.

In a third specific example of the complexes of the invention, the gallium-ligand complex is a complex of ionic gallium with (S)-(+) mandelic acid (C₂₄H₂₁GaO₉). A proposed structure for this complex may be represented by structural Formula (III):

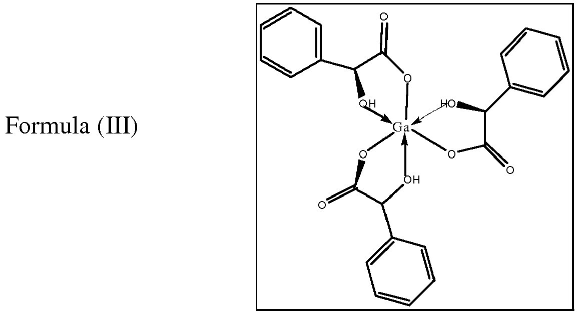

The gallium (S)-(+) mandalate complex is a neutral complex with a molecular weight of 523, which supports a conclusion of formation of a 3:1 complex. The gallium (S)-(+) mandalate complex contains about 6-16% gallium, about 7-14% gallium, about 10% gallium or about 8% gallium, resulting in a high proportion of gallium relative to the molecular weight of the complex.

In a fourth specific example of the complexes of the invention, the gallium-ligand complex is a complex of ionic gallium with (R)-(-) mandelic acid (C₂₄H₂₁GaO₉). A proposed structure for this complex may be represented by structural Formula (IV):

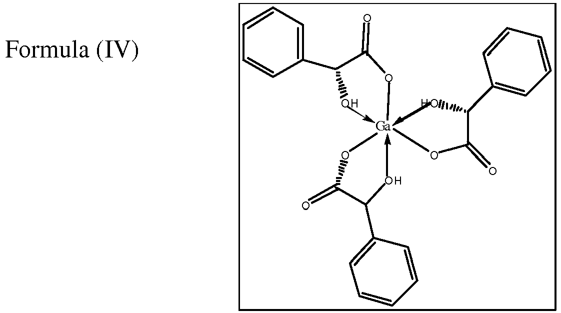

The gallium (R)-(-) mandalate complex is a neutral complex with a molecular weight of 523, which supports a conclusion of formation of a 3:1 complex. The gallium (R)-(-) mandalate complex contains about 6-16% gallium, about 7-14% gallium, about 10% gallium or about 8% gallium, resulting in a high proportion of gallium relative to the molecular weight of the complex.

In a fifth specific example of the complexes of the invention, the gallium-ligand complex is a neutral complex of ionic gallium with histidine. A proposed structure for this complex may be represented by structural Formula (V):

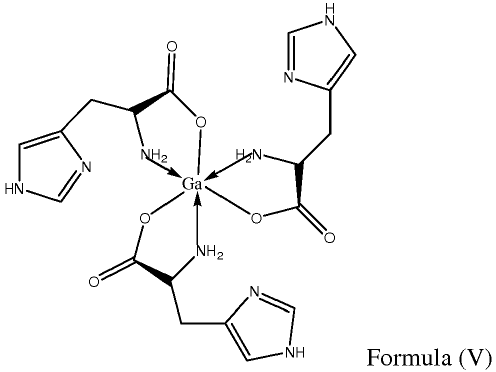

The neutral complex of gallium with histidine has a molecular weight of 532, which supports a conclusion of formation of a 3:1 complex

In a sixth specific example of the complexes of the invention, the gallium-ligand complex is an ionic complex of ionic gallium with histidine. For example, if gallium nitrate is used to prepare the complex, the complex of this embodiment may be represented by the formula C₁₈H₂₇GaN₉O₆((NO₃)₃), a nitrate salt. In a similar fashion one would get different salts, such as C₁₅H₂₄GaN₃O₆(X₃); wherein X is Cl, I, or sulfate depending what gallium salt is used for the preparation. A proposed structure for this complex may be represented by structural Formula (VI): 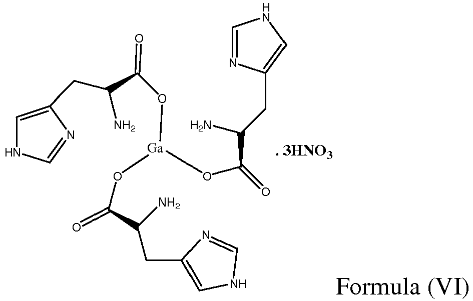 Or, alternatively, by structural Formula (VIa)

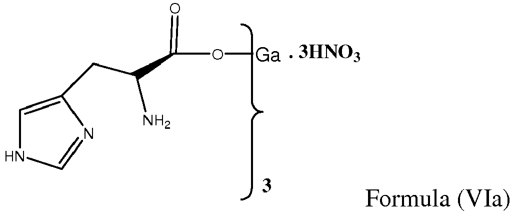

The ionic complex of gallium and histidine has a molecular weight of 535 in its non-salt form, which supports a conclusion of formation of a 3:1 complex.

In a seventh specific example of the complexes of the invention, the gallium-ligand complex is a complex of ionic gallium with carnosine. A proposed structure for this complex may be represented by structural Formulae (VII) or, alternatively, by structural Formula (VIIa):

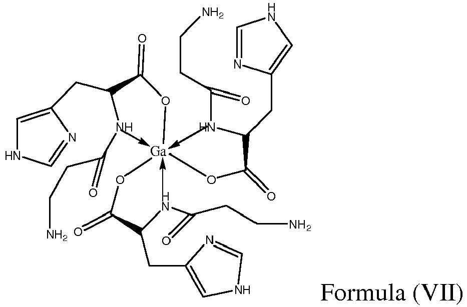

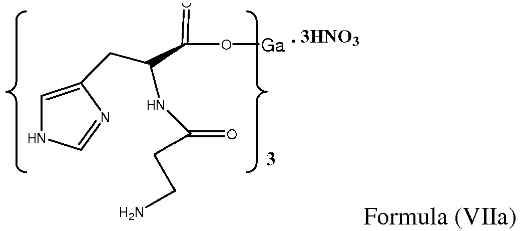

The gallium complex with carnosine is a neutral complex with a molecular weight of 745, which supports a conclusion of formation of a 3:1 complex. The gallium content of the complex is about 9.35%.

In another aspect, the invention provides pharmaceutical compositions comprising the gallium-ligand complexes described above and at least one pharmaceutically acceptable excipient, and methods of making such pharmaceutical compositions. More particularly, embodiments of the present invention provide novel pharmaceutical compositions comprising the gallium-ligand complexes which are formulated in dosage forms suitable for oral administration. Pharmaceutical compositions in a form for oral administration include tablets, capsules, caplets, etc. The particular pharmaceutical excipient(s) chosen to make such pharmaceutical compositions and oral dosage forms, as well as other delivery forms of the composition, will depend at least in part on the desired administration route. Examples of pharmaceutically acceptable excipients and methods of manufacture for various compositions, and dosage or delivery forms are well known in the art and may be found in, e.g., Remington's Pharmaceutical Sciences, (Gennaro ed., 20th ed. 2000).

For example, when used for oral administration the gallium-ligand complexes and pharmaceutical compositions described herein will typically be formulated as a solid tablet, capsule or caplet incorporating one or more solid excipients, such as, e.g., starch, lactose, dextran, magnesium stearate, microcrystalline cellulose, methyl cellulose, hydroxypropyl cellulose, Croscarmellose, crospovidone, sodium starch glycolate, silicon dioxide, hydroxypropyl methylcellulose, sucrose, glucose, mannitol, sorbitol, calcium carbonate, cyclodextrins, etc. In this regard, the gallium-ligand complexes disclosed herein will generally be produced in the form of a powder or wet cake which may be mixed with desired excipients and granulated or directly compressed into tablets.

A tableted pharmaceutical composition comprising the gallium-ligand complexes of the invention will preferably have an oral bioavailability profile (e.g., Peak [Ga]ₚₗₐₛₘₐ; Time to Peak [Ga]ₚₗₐₛₘₐ; etc.) similar to that for IV administration of an equal amount of gallium. A gallium-ligand tablet containing 30 mg gallium may provide peak [Ga]ₚₗₐₛₘₐ of between about 0.1 and 10 µg/mL or between about 1,000 and 3,000 ng/mL at a time of between about 30 and 60 minutes. Tablets may also contain less than about 30 mg gallium, up to about 150 mg gallium or up to about 300 mg gallium. These tablets may provide peak [Ga]ₚₗₐₛₘₐ of between about 0.2 and 3 µg/mL at a time of between about 15 and 120 minutes.

The pharmaceutical compositions comprising the gallium-ligand complexes can also be in the form of a solution, suspension or emulsion incorporating a liquid excipient, such as, e.g., water, propylene glycol, polyethylene glycol, sorbitol, maltitol, sucrose or a pharmaceutically acceptable buffer, such as phosphate, citrate or carbonate buffer. Such pharmaceutical compositions may be adapted for systemic or local administration.

In yet another aspect, the invention provides methods for preventing or treating conditions and diseases such as cancer, hypercalcemia, osteoporosis, osteopenia, bone fracture, Paget's disease (i.e., any bone disorder mediated by the action of osteoclasts and osteoblasts or tumor cells), arthritis, navicular disease (for example, in horses) autoimmune diseases, local or generalized inflammatory diseases, and bacterial infections using the gallium-ligand complexes and pharmaceutical compositions described above. The methods comprise administering, to a subject suffering from a disease which is treatable of preventable by gallium, a therapeutically effective amount of a pharmaceutical composition comprising the gallium-ligand complexes of the invention. In the case of treatment of bacterial infections, the gallium-ligand complexes, pharmaceutical compositions and dosage forms may optionally be administered in combination with conventional antibacterials and antibiotics. Doses are selected to provide pharmaceutically active plasma gallium concentrations for the treatment of the disease or condition, which in the case of excessive resorption of calcium from bone (e.g., arising from cancer, hypercalcemia, osteoporosis, osteopenia, Paget's disease, etc.) is established to be about 0.1-20.0 µg/ml, preferably about 0.5-2.0 µg/ml. Such blood levels may be achieved by administering about 0.1-2.0 grams of gallium daily. For the treatment of various forms of cancer, including cancer-related hypercalcemia, gallium is typically administered in the range from about 0.25 mg/kg/day to about 10 mg/kg/day, preferably from about 0.5 mg/kg/day to about 5 mg/kg/day, from which the administration of the gallium-ligand complexes of the present invention can be extrapolated. Such doses may be administered as a single unit dose or in a number of smaller doses.

Other dosage regimens for gallium are known to those skilled in the art (see, e.g., Physician's Desk Reference, 58th ed. (2004)). Dosages may be varied depending upon the requirements of the patient and the severity of the condition being treated. The amount and frequency of administration will be regulated according to the judgment of the attending clinician considering such factors as age, condition and size of the patient as well as severity of the symptoms being treated.

A further aspect of the invention provides for use of any of the gallium-ligand complexes, and pharmaceutical compositions and dosage forms comprising them, as medicaments. In a specific aspect the gallium-ligand complexes, and pharmaceutical compositions and dosage forms comprising them, are used for the treatment of cancer, hypercalcemia, osteoporosis, osteopenia, Paget's disease, any bone disorder characterized by bone resorption which is mediated by the action of osteoclasts and osteoblasts or tumor cells, or bacterial infections as described above.

A further embodiment of the present invention relates to methods for the preparation of the gallium-ligand complexes described herein. The gallium salt used to form the complex with the ligand may be any gallium salt, including, but not limited to, gallium iodide, gallium chloride, and gallium sulfate and gallium nitrate. The ligand selected for complexing with ionic gallium contributed by the gallium salt may be any of the following:
an amino acid or amino acid derivative, including naturally occurring or a non-naturally occurring analogs of α and β amino acids, including histidine, carnosine and methionine;
a mono or poly carboxylic acid or carboxylic acid derivative, including aliphatic, aromatic and heterocyclic carboxylic acids, and pyridine compounds such as picolinic acid (C₆H₅NO₂, pyridine-2-carboxylic acid);
an aromatic, aliphatic or heterocyclic α-hydroxy acid, including mandelic acid (C₆H₅CH(OH)CO₂H, 2-hydroxy-2-phenylacetic acid); or
an α-hydroxypyrone, including kojic acid (C₆H₆O₄; 5-hydroxy-2-(hydroxymethyl)-4-pyrone), hydroxymethyl-protected kojic acid and isomaltol.

In specific embodiments for preparation of the gallium-ligand complexes, the ligand for complexing with ionic gallium contributed by the gallium salt may be selected from the group consisting of histidine, methionine, picolinic acid, mandelic acid, kojic acid, hydroxymethyl-protected kojic acid, isomaltol, carnosine, and 2, 3-Dihydro-3, 5-dihydroxy-6-methyl*-4h-pyran-4-one.*

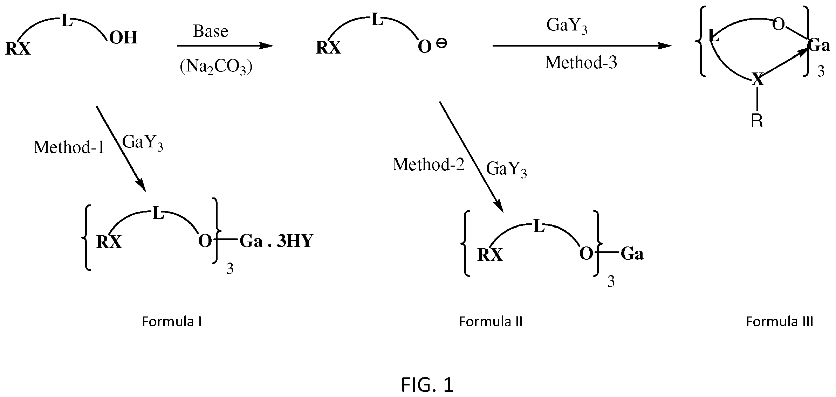

As examples, three types of general procedures can be employed to make the gallium- ligand complexes, as illustrated in Fig. 1. As shown in the Fig. 1, RX-L-OH is a poly-dentate (e.g., bi-dentate, tri-dentate or tetra-dentate) molecule capable of forming cyclic, salt/coordination complexes with gallium. R, X, and L are part of the ligand, and L is the portion of the ligand molecule that is not RX or OH. OH can be a hydroxyl, phenol, or part of a carboxylic acid. X is any atom capable of coordinating electrons, including elements such as O, N, S, etc. R can be hydrogen, alkyl, aryl and acyl. Y is the counter ion on gallium (e.g., chloride, nitrate, sulfate, etc.).

Method-1 involves heating the inorganic gallium salt GaY₃ (e.g., Y = bromide, chloride, or nitrate) with an organic acidic ligand (RX-L-OH) in a solution of alcohol, water, or a mixture thereof for a few hours and isolating the formed gallium-organic ligand complex. If the organic ligand contains basic nitrogen, the product is usually formed as a salt 3HY (HNO₃, HCl, HBr etc.). The end product is isolated by a suitable method such as crystallization, lyophilization, evaporation, extraction etc. The product may have cyclic or non-cyclic structure.

Method-2 involves heating the inorganic gallium salt GaY₃ (e.g., Y = bromide, chloride, or nitrate) with an organic acidic ligand RX-L-OH in a solution of alcohol, water, or a mixture thereof in the presence of a base such as sodium carbonate, bicarbonate, etc. for a few hours and isolating the formed gallium-organic ligand complex. In this case the product is usually isolated as the neutral complex. The product may have a cyclic or non-cyclic structure. The end product is isolated by a suitable method such as crystallization, lyophilization, evaporation, extraction, etc.

Method-3 involves pre-making the inorganic salt of the organic acidic ligand RX-L-OH using a base such as sodium carbonate, bicarbonate, etc. followed by heating with the inorganic gallium salt GaY₃ (e.g., Y = bromide, chloride, or nitrate) in a solution of alcohol, water, or a mixture thereof with or without additional base such as sodium carbonate, bicarbonate, etc. for a few hours and isolating the formed gallium-organic ligand complex. In this case the product is usually isolated as the neutral complex. The product may have cyclic or non-cyclic structure. The end product is isolated by a suitable method such as crystallization, lyophilization, evaporation, extraction etc.

As a further example, the gallium-ligand complexes are prepared using a modification of the procedure disclosed in U.S. Patent No. 7,119,217. In this process a solution of the selected sodium carboxylate ligand is prepared in alcohol, for example ethanol, using a minimum volume of water at 50-100 °C. The selected gallium salt, for example Ga(NO₃)₃, is added to the anion solution in portions. The reaction is allowed to proceed for a period of time sufficient for the desired gallium-ligand complexes to form. Depending on the complex being prepared, the gallium-ligand complex may precipitate spontaneously from the alcohol/water solution. Other complexes may require removal of the alcohol under vacuum and filtration to recover the complexes. The collected solids are generally washed with water to remove residual counterions contributed by the gallium salt and the carboxylic acid followed by washing with organic solvents to remove any free ligands.

Alternatively, the ionic gallium-ligand complexes of the invention may be prepared by a method based on the preparation of chromium nitrate complexes as described by Oki, H. Bulletin of the Chemical Society of Japan. 1977. 50(3), 680-684. This procedure is expected to be most useful for preparation of ionic complexes where the counterion of the gallium salt is pharmaceutically acceptable, for example using GaCl₃.

### EXAMPLES

### 1. Preparation of Neutral Gallium Complexes (Exemplified by Picolinic Acid Complex)

Picolinic acid (6.18 g, 50.20 mmol, 3.07 eq) was suspended in ethanol (absolute, 125 mL). The mixture was heated to 70°C to provide a homogeneous solution. To the heated solution was added water (1 mL) then Na₂CO₃ (2.66 g, 25.10 mmol, 1.54 eq), in portions (CO₂ evolution). The mixture was heated for 1 h at 70°C. As the sodium carboxylate formed the solution became a viscous mixture. Following 1 h of heating, water (5 mL) was added to provide a homogenous solution. After the addition of water, Ga(NO₃)₃ (4.18 g, 16.35 mmol, 1.0 eq) was added in portions to the heated mixture. The homogeneous solution was heated for 2 h then heat was removed (solids became evident as the mixture cooled). The mixture was stirred at ambient temperature for 12 h. The heterogeneous mixture was then filtered, the collected solids were washed with water (25 mL x 2). The solids were then dried in a vacuum oven (50C) for 16 h. This provided 3.5 g of the gallium complex.

The picolinic acid complex was characterized as follows:
a. 1H NMR (400 MHz, DMSO-d6): 8.74-8.63 (m, 1H), 8.45 (m, 3H), 8.00-7.61 (m 2 H)
b. FTIR: 3526, 3449, 3079, 1683, 1668, 1607, 1569, 1472, 1452, 1363, 1333, 1290, 1240, 1162, 1099, 1049, 1029, 918, 856, 833, 766, 712, 693, 652, 540.
c. MS Data (M+H): 436.0
d. Gallium analysis: 14.51%
e. C,H,N Analysis:
   Found: C 48.20%, H 2.70%, N 9.33%
f. Karl Fischer (H₂O wt%): 1.69%
g. Nitrate analysis:
   Found: 0.36%

The analytical data for the picolinic acid complex closely matched the characteristics calculated/predicted for a 1:3 (Ga:ligand complex). Residual sodium nitrate contamination was minimal (0.36% nitrate) and the proportion of gallium in the complex was particularly high in relation to the molecular weight of the complex (15.99% without solvents).

### 2. Preparation of Additional Neutral Gallium-Ligand Complexes

Complexes of gallium with N-acyl piperidinic acid, (+)-mandelic acid, (-) mandelic acid, kojic acid and tryptophan were prepared essentially as described in (1) above and characterized as follows:

Characterization of N-acetyl piperidinic acid complex:
a. 1H NMR (400 MHz, DMSO-d6): 4.93 (m, 1H), 4.24 (m, 1H), 3.63 (d, 1 H), 3.25 (t, 1H), 2.59 (t, 1H), 2.15 (dd, 1H), 1.99 (s, 3 H), 1.90 (s, 3 H), 1.60-1.22 (m, 9 H)
b. FTIR: 2934, 2861, 1716, 1616, 1425, 1260, 1220, 1167, 1143, 1035, 996, 967, 935, 914, 866, 814, 780, 719
c. MS Data (M+H): 580.2
d. Gallium analysis: 7.79%
e. C,H,N Analysis:
   Found: C 44.37, H 5.68%, N 6.33%
f. Karl Fischer (H₂O wt%): 1.10%

The analytical data for the acetyl piperidinate complex closely matched the characteristics calculated/predicted for a 1:3 (Ga:ligand complex). The proportion of gallium in the complex was high in relation to the molecular weight of the complex (12.01% without solvents).

Characterization of (+)-mandelic acid complex:
a. 1H NMR (400 MHz, DMSO-d6): 7.40 (m, 2H), 7.31 (m, 2H), 7.26 (m, 1H), 7.15-7.06 (m 2H), 7.00 (m, 3H), 4.90 (s, 1H), 4.39 (s, 2H)
b. FTIR: 3034, 1739, 1694, 1644, 1496,1455, 1331, 1185, 1094, 1075, 1020, 934, 817, 726, 694, 619, 605.
c. MS Data (M+Na): 545.0
d. Gallium analysis: 8.02%
e. C,H,N Analysis:
   Found: C 53.55, H 4.21%
f. Karl Fischer (H₂O wt%): 2.93%
g. Nitrate analysis:
   Found: 0.72%

The analytical data for the (+)-mandalate complex closely matched the characteristics calculated/predicted for a 1:3 (Ga:ligand complex). Residual sodium nitrate contamination was minimal (0.72% nitrate) and the proportion of gallium in the complex was high in relation to the molecular weight of the complex (13.33% without solvents).

Characterization of (-)-mandelic acid complex:
a. 1H NMR (400 MHz, DMSO-d6): 7.40 (m, 2H), 7.31 (m, 2H), 7.26 (m, 1H), 7.15-7.06 (m 2H), 7.00 (m, 3H), 4.90 (s, 1H), 4.39 (s, 2H)
b. FTIR: 3034, 1739, 1693, 1644, 1496,1455, 1333, 1185, 1094, 1075, 1022, 945, 817, 727, 619, 605.
c. MS Data (M+Na): 545.0
d. Gallium analysis: 8.02%
e. C,H,N Analysis:
   Found: C 53.43, H 3.82%
f. Karl Fischer (H₂O wt%): 3.10%
g. Nitrate analysis:
   Found: 0.52%

The analytical data for the (-)-mandalate complex closely matched the characteristics calculated/predicted for a 1:3 (Ga:ligand complex). Residual sodium nitrate contamination was minimal (0.52% nitrate) and the proportion of gallium in the complex was high in relation to the molecular weight of the complex (13.33% without solvents).

Characterization of the kojic acid complex:
a. 1H NMR (400 MHz, DMSO-d6): 8.15 (s, 1H), 6.76 (m, 1H), 4.43 (s, 4H), 1.71 (s, 1H)
b. FTIR: 3321, 1615, 1559, 1515, 1464, 1274, 1251, 1203, 1185, 1152, 1079, 1038, 981, 945, 921, 871, 816, 795, 760, 635, 607, 568, 539
c. MS Data (M+Na): 515.8
d. Gallium analysis: 11.13%
e. C,H,N Analysis:
   Found: C 37.09, H 2.42%
f. Karl Fischer (H₂O wt%): 0.26%
g. Nitrate analysis:
   Found: 4.72%

The analytical data for the kojate complex closely matched the characteristics calculated/predicted for a 1:3 (Ga:ligand complex). Residual sodium nitrate contamination was minimal (4.72% nitrate) and the proportion of gallium in the complex was high in relation to the molecular weight of the complex (14.14% without solvents).

Characterization of the neutral proline complex:
a. 1H NMR(400 MHz, DMSO-d6):: 3.60-3.00 (m, 5 H), 1.95 (m, 1H), 1.66 (m, 1H)
b. FTIR: 3208, 2980, 2875, 1656, 1456, 1355, 1324, 1296, 1267, 1191, 1171, 1103, 1072, 1048, 987, 966, 933, 920, 887, 847, 811, 787, 705, 620, 574
c. MS Data (M+): 412
d. Gallium analysis: 8.97%
e. C,H,N Analysis:
   Found: C 26.12%, H 3.00%, N 11.64%
f. Karl Fischer (H₂O wt%): 0.19%
g. Nitrate analysis
   Found: 28.43%

The analytical data for the neutral proline complex closely matched the characteristics calculated/predicted for a 1:3 (Ga:ligand) complex. Residual sodium nitrate contamination was higher than in other complexes, but not unacceptable (28.43% nitrate) and the proportion of gallium in the complex was particularly high in relation to the molecular weight of the complex (16.92% without solvents).

Characterization of the tryptophan complex:
a. 1H NMR(400 MHz, DMSO-d6):: 10.97-10.86 (m, 1H), 7.54-7.39 (m, 2H), 7.36-6.97 (m, 3H), 5.04 - 4.18 (m, 1H), 3.70-3.20 (m, 3 H), 3.10 - 2.71 (m, 1H)
b. FTIR: 3210, 2981, 2871, 1647, 1450, 1355, 1322, 1291, 1264, 1181, 1157, 1103, 1072, 1048, 987, 968, 916, 881, 847, 811, 787, 705,
c. MS Data (M+): 679.4
d. Gallium analysis: 7.86%
e. C,H,N Analysis:
   Found: C 54.25%, H 4.4%, N 11.73%
f. Karl Fischer (H₂O wt%): 4.09%
g. Nitrate analysis
   Found: 1.42%

The analytical data for the tryptophan complex closely matched the characteristics calculated/predicted for a 1:3 (Ga:ligand complex). Residual sodium nitrate contamination was minimal (1.42% nitrate) and the proportion of gallium in the complex was high in relation to the effective molecular weight of the complex (10.26% without solvents).

### 3. Preparation of Ionic Gallium Complexes (Exemplified by Proline Complex)

Ga(NO3)3 (500 mg, 1.96 mmol, 1.0 eq) and L-proline (676 mg, 5.87 mmol, 3.0 eq)) were combined in water (5 mL). The mixture was heated, with stirring, for 18 h. The homogenous mixture was then lyophilized to provide the Ga(L-proline)-(HNO₃)3 (1.13 g).

Characterization of the ionic proline complex:
a. 1H NMR(400 MHz, D₂O):: 4.17 (m, 1 H), 3.42-3.28 (m, 2H), 2.33 (m, 1H), 2.09-1.92 (m 3H)
b. FTIR: 2984, 1733, 1622, 1302, 1090, 1037, 988, 937, 857, 825, 666, 574, 542
c. MS Data (M+H): 415
d. Gallium analysis: 7.66%
e. C,H,N Analysis:
   Found: C 34.02%, H 5.48%, N 12.70%
f. Karl Fischer (H₂O wt%): 5.89%
g. Nitrate analysis
   Found: 23.35%

The analytical data for the ionic proline complex closely matched the characteristics calculated/predicted for a 1:3 (Ga:ligand complex). Residual nitrate ion was present as the nitric acid salt at a level of 23.35% nitrate and the proportion of gallium in the complex was high in relation to the effective molecular weight of the complex (11.6% without solvents).

### 4. Oral Bioavailability of Gallium Complexes

Pharmacokinetics, oral bioavailability and bioequivalency of gallium-ligand complexes according to the invention were determined in dogs following a single oral gavage administration of the compounds as a suspension/solution in water in fasted male Beagle dogs at 2.5 mg/kg dog body weight or following administration of a single total dose of 30 mg in a capsule formulation (for histidine and carnosine only). The following complexes were selected for testing: gallium picolinate, gallium kojate, gallium (+)-mandalate, gallium (-)-mandalate, gallium tryptophanate, gallium acetyl piperidinate, gallium prolinate both neutral and ionic; and the capsules prepared from histidine and carnosine. Each compound was studied in groups of three fasted animals. Nine serial blood samples were collected from each animal by venipuncture of a jugular vein at the following time points post-dose: 0 (predose), 0.25, 0.5, 0.75, 1, 2, 4, 8, and 16 hours post-dose. Blood samples were collected into chilled tubes containing heparin as the anticoagulant. Plasma from these blood samples was analyzed for gallium concentration by ICP-MS to demonstrate oral bioavailability and pharmacokinetics comparable to intravenous administration of gallium. The results of the test gallium complexes were compared with oral bioavailability of gallium maltolate (the positive control).

Oral bioavailability of the test gallium complex was evaluated in each of the three animals in each test group. The gallium maltolate positive control reached blood levels of ≥1,000 ng/ml in all three dogs in the group. The gallium nitrate oral negative control did not achieve greater than 700 ng/ml gallium in blood (one animal). Therefore, the criterion for acceptability of the test gallium complex was a peak blood concentration of gallium that was at least approximately 1,000 ng/ml in at least two animals in the test group for that complex.

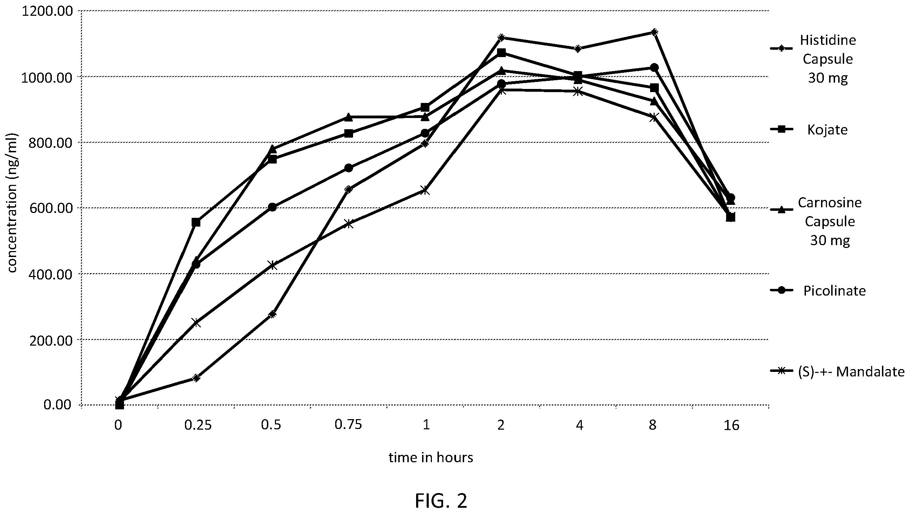

Of the gallium test complexes evaluated, gallium picolinate, gallium kojate, gallium (+)-mandalate, gallium (-)-mandalate, and the capsules prepared from histidine and carnosine reached blood levels at least about 1,000 ng/ml in two of the three animals in the test group. The results are shown in Fig. 2, which is a graph of the average blood concentration of gallium in each of these test groups after eliminating the data for the outlier (i.e., the third animal which did not achieve at least about 1,000 ng/ml). These complexes were therefore identified as promising complexes for oral delivery of bioavailable gallium, comparable to gallium maltolate.

In contrast, the maximum blood concentration achieved using the gallium prolinate nitrate salt was about 200 ng/ml, the maximum blood concentration achieved using the gallium prolinate neutral complex was about 300 ng/ml, the maximum blood concentration achieved using the gallium tryptophanate complex was about 500 ng/ml, and the maximum blood concentration achieved using the gallium acetyl piperidinate complex was about 200 ng/ml, which was considered too low to be practical for oral administration.

These results were unexpected due to the substantial similarity between the complexes that exhibited high oral bioavailability and those that exhibited unacceptably low oral bioavailability. That is, proline, tryptophan and N-acetyl piperidinic acid have a striking structural similarity to the ligands of the highly bioavailable complexes (histidine, kojic acid, carnosine, mandelic acid and picolinic acid), were shown to form the same 3:1 complexes with gallium, and also contained high proportions of gallium relative to the molecular weight of the complex. To illustrate this point, the tryptophanate, prolinate and acetyl piperidinate gallium complexes are discussed below.

The complex of ionic gallium with tryptophan (C₃₃H₃₃GaN₆O₆) has a proposed structure which may be represented as follows:

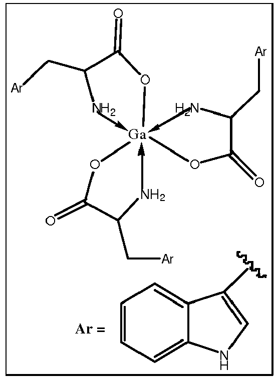

The gallium-tryptophan complex is a neutral complex with a calculated effective molecular weight of 679, which supports a conclusion of formation of a 3:1 complex, and contained a high proportion of gallium relative to the molecular weight of the complex.

The complex of ionic gallium with proline was formed as a nitrate salt (C₁₅H₂₄GaN₃O₆((NO₃)₃) and has a proposed structure which may be represented as follows: 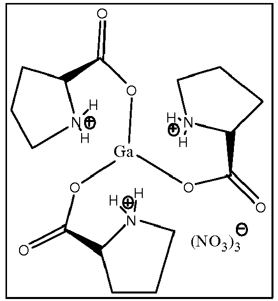 or, alternatively,

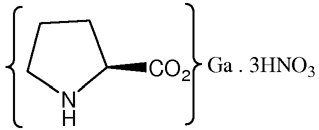

The ionic gallium prolinate complex has a molecular weight of 412 in its non-salt form, which supports a conclusion of formation of a 3:1 complex, and contained a high proportion of gallium relative to the molecular weight of the complex.

The complex of ionic gallium with N-acetyl piperidinic acid (C₂₄H₃₆GaN₃O₉) has a proposed structure which may be represented as follows:

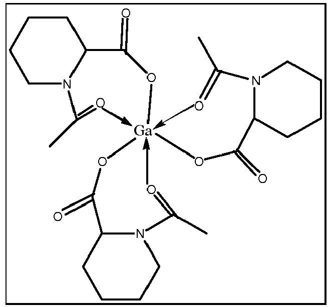

The gallium acetyl piperidinate complex is a neutral complex with a molecular weight of 580, which supports a conclusion of formation of a 3:1 complex, and contained a high proportion of gallium relative to the molecular weight of the complex.

Based on these experimental results, it is concluded that the availability of oxygen and/or nitrogen moieties in the ligand, and the ability to form a 3:1 complex with gallium, is not predictive of utility as an agent for oral delivery of gallium or of gallium oral bioavailability.

Reference throughout this specification to "one embodiment," "certain embodiments," "one or more embodiments" or "an embodiment" means that a particular feature, structure, material, or characteristic described in connection with the embodiment is included in at least one embodiment of the invention. Thus, the appearances of the phrases such as "in one or more embodiments," "in certain embodiments," "in one embodiment" or "in an embodiment" in various places throughout this specification are not necessarily referring to the same embodiment of the invention. Furthermore, the particular features, structures, materials, or characteristics may be combined in any suitable manner in one or more embodiments.

Although the invention herein has been described with reference to particular embodiments, it is to be understood that these embodiments are merely illustrative of the principles and applications of the present invention. Thus, it is intended that the present invention include modifications and variations that are within the scope of the appended claims.

## Claims

1. A compound comprising ionic gallium in a complex with a ligand selected from the group consisting of histidine, carnosine and mandelic acid, and wherein the ratio of ligand to ionic gallium in the complex is 3:1.

2. The compound according to claim 1 which is gallium (+)-mandelate, gallium (-)-mandelate, gallium histidinate or gallium carnosinate.

3. The compound according to claim 1 or 2 which is a neutral complex.

4. The compound according to claim 1 or 2 which is ionic gallium histidinate or gallium carnosinate.

5. The compound according to any one of claims 1 to 4 which further comprises a counterion selected from the group consisting of nitrate, chloride, bromide, iodide, and sulfate.

6. The compound according to any one of claims 1 to 5, wherein nitrate is present in an amount of no more than 5% or in an amount of 0.3 to 2%.

7. A pharmaceutical composition for use in therapy, comprising a compound comprising ionic gallium in a complex with a ligand selected from the group consisting of picolinic acid, kojic acid, mandelic acid, (+)-mandelic acid, (-)-mandelic acid, histidine and carnosine, wherein the ratio of ligand to ionic gallium in the complex is 3:1 and at least one pharmaceutically acceptable excipient.

8. The pharmaceutical composition for use
according to claim 7 wherein the compound is gallium picolinate or gallium kojate.

9. The pharmaceutical composition for use
according to claim 7 or 8 which is a neutral complex.

10. The pharmaceutical composition for use according
to claim 7 which is ionic gallium histidinate or gallium carnosinate.

11. The pharmaceutical composition for use
according to any one of claims 7 to 10 which further comprises a counterion selected from the group consisting of nitrate, chloride, bromide, iodide, and sulfate.

12. The pharmaceutical composition for use
according to any one of claims 7 to 11, wherein nitrate is present in an amount of no more than 5% or in an amount of 0.3 to 2%.

13. The pharmaceutical composition for use
according to any one of claims 7 to 12, wherein the gallium complex is present in a crystalline form.

14. The pharmaceutical composition for use
according to any one of claims 7 to 13 which is formulated for oral administration.

15. The pharmaceutical composition for use
according to any one of claims 7 to 14 which is in the form of a tablet, capsule or caplet, powder, solution or suspension.

16. The pharmaceutical composition for use
according to any one of claims 7 to 15 in the treatment of bacterial infection, cancer, hypercalcemia, osteoporosis, osteopenia, arthritis, autoimmune disease, or Paget's disease.

17. The pharmaceutical composition for use
according to any one of claims 7 to 15 in the treatment of bacterial infection in combination with an antibiotic or antibacterial compound.

## Patentansprüche

1. Eine Verbindung, umfassend ionisches Gallium in einem Komplex mit einem Liganden, ausgewählt aus der Gruppe bestehend aus Histidin, Carnosin und Mandelsäure, und wobei das Verhältnis von Ligand zu ionischem Gallium in dem Komplex 3:1 beträgt.

2. Die Verbindung nach Anspruch 1, bei welcher es sich um Gallium(+)-mandelat, Gallium(-)-mandelat, Galliumhistidinat oder Galliumcarnosinat handelt.

3. Die Verbindung nach Anspruch 1 oder 2, welche ein neutraler Komplex ist.

4. Die Verbindung nach Anspruch 1 oder 2, bei welcher es sich um ionisches Galliumhistidinat oder Galliumcarnosinat handelt.

5. Die Verbindung nach einem der Ansprüche 1 bis 4, welche weiter ein Gegenion, ausgewählt aus der Gruppe bestehend aus Nitrat, Chlorid, Bromid, Iodid und Sulfat, umfasst.

6. Die Verbindung nach einem der Ansprüche 1 bis 5, wobei Nitrat in einer Menge von nicht mehr als 5% oder in einer Menge von 0,3 bis 2% vorliegt.

7. Ein Arzneimittel zur Verwendung in der Therapie, umfassend eine Verbindung, die ionisches Gallium in einem Komplex mit einem Liganden, ausgewählt aus der Gruppe bestehend aus Picolinsäure, Kojisäure, Mandelsäure, (+)-Mandelsäure, (-)-Mandelsäure, Histidin und Carnosin, umfasst, wobei das Verhältnis von Ligand zu ionischem Gallium in dem Komplex 3:1 beträgt, und mindestens einen pharmazeutisch verträglichen Exzipienten.

8. Das Arzneimittel zur Verwendung nach Anspruch 7, wobei die Verbindung Galliumpicolinat oder Galliumkojat ist.

9. Das Arzneimittel zur Verwendung nach Anspruch 7 oder 8, bei welchem es sich um einen neutralen Komplex handelt.

10. Das Arzneimittel zur Verwendung nach Anspruch 7, bei welchem es sich um ionisches Galliumhistidinat oder Galliumcarnosinat handelt.

11. Das Arzneimittel zur Verwendung nach einem der Ansprüche 7 bis 10, welches weiter ein Gegenion, ausgewählt aus der Gruppe bestehend aus Nitrat, Chlorid, Bromid, Iodid und Sulfat, umfasst.

12. Das Arzneimittel zur Verwendung nach einem der Ansprüche 7 bis 11, wobei Nitrat in einer Menge von nicht mehr als 5% oder in einer Menge von 0,3 bis 2% vorliegt.

13. Das Arzneimittel zur Verwendung nach einem der Ansprüche 7 bis 12, wobei der Galliumkomplex in kristalliner Form vorliegt.

14. Das Arzneimittel zur Verwendung nach einem der Ansprüche 7 bis 13, welches zur oralen Verabreichung formuliert ist.

15. Das Arzneimittel zur Verwendung nach einem der Ansprüche 7 bis 14, welches in Form einer Tablette, Kapsel oder kapselförmigen Tablette, eines Pulvers, einer Lösung oder Suspension vorliegt.

16. Das Arzneimittel zur Verwendung nach einem der Ansprüche 7 bis 15 bei der Behandlung von bakterieller Infektion, Krebs, Hyperkalzämie, Osteoporose, Osteopenie, Arthritis, Autoimmunerkrankung oder Morbus Paget.

17. Das Arzneimittel zur Verwendung nach einem der Ansprüche 7 bis 15 bei der Behandlung von bakterieller Infektion in Kombination mit einer antibiotischen oder antibakteriellen Verbindung.

## Revendications

1. Composé comprenant du gallium ionique dans un complexe avec un ligand sélectionné dans le groupe constitué par l'histidine, la carnosine et l'acide mandélique, et dans lequel le rapport entre le ligand et le gallium ionique dans le complexe est de 3:1.

2. Composé selon la revendication 1 qui est le mandélate-(+) de gallium, le mandélate-(-) de gallium, l'histidinate de gallium ou le carnosinate de gallium.

3. Composé selon la revendication 1 ou 2 qui est un complexe neutre.

4. Composé selon la revendication 1 ou 2 qui est l'histidinate de gallium ionique ou
le carnosinate de gallium.

5. Composé selon l'une quelconque des revendications 1 à 4 qui comprend en outre
un contre-ion sélectionné dans le groupe constitué par le nitrate, le chlorure, le bromure, l'iodure, et le sulfate.

6. Composé selon l'une quelconque des revendications 1 à 5, dans lequel le nitrate est présent en une quantité qui n'excède pas 5 % ou en une quantité de 0,3 à 2 %.

7. Composition pharmaceutique pour une utilisation en thérapie, comprenant un composé comprenant du gallium ionique dans un complexe avec un ligand sélectionné dans le groupe constitué par l'acide picolinique, l'acide kojique, l'acide mandélique, l'acide mandélique-(+), l'acide mandélique-(-), l'histidine et la carnosine, dans laquelle le rapport entre le ligand et le gallium ionique dans le complexe est de 3:1 et au moins un excipient acceptable sur le plan pharmaceutique.

8. Composition pharmaceutique pour une utilisation selon la revendication 7 dans
laquelle le composé est le picolinate de gallium ou le kojate de gallium.

9. Composition pharmaceutique pour une utilisation selon la revendication 7 ou 8,
qui est un complexe neutre.

10. Composition pharmaceutique pour une utilisation selon la revendication 7 qui est l'histidinate de gallium ionique ou le carnosinate de gallium.

11. Composition pharmaceutique pour une utilisation selon l'une quelconque des revendications 7 à 10 qui comprend en outre un contre-ion sélectionné dans le groupe constitué par le nitrate, le chlorure, le bromure, l'iodure, et le sulfate.

12. Composition pharmaceutique pour une utilisation selon l'une quelconque des revendications 7 à 11, dans laquelle le nitrate est présent en une quantité qui n'excède pas 5 % ou en une quantité de 0,3 à 2 %.

13. Composition pharmaceutique pour une utilisation selon l'une quelconque des revendications 7 à 12, dans laquelle le complexe de gallium est présent sous une forme cristalline.

14. Composition pharmaceutique pour une utilisation selon l'une quelconque des revendications 7 à 13 qui est formulée pour l'administration orale.

15. Composition pharmaceutique pour une utilisation selon l'une quelconque des revendications 7 à 14 qui est sous la forme d'un comprimé, d'une gélule ou d'un cachet, d'une poudre, d'une solution ou d'une suspension.

16. Composition pharmaceutique pour une utilisation selon l'une quelconque des revendications 7 à 15 dans le traitement des infections bactériennes, du cancer, de l'hypercalcémie, de l'ostéoporose, de l'ostéopénie, de l'arthrite, des maladies auto-immunes ou de la maladie de Paget.

17. Composition pharmaceutique pour une utilisation selon l'une quelconque des revendications 7 à 15 dans le traitement des infections bactériennes en combinaison avec un composé antibiotique ou antibactérien.
